**Europäisches Patentamt**

**European Patent Office**

**Office européen des brevets**

(19)

(11) Veröffentlichungsnummer : **0 384 212 B1**

## (12) EUROPÄISCHE PATENTSCHRIFT

(45) Veröffentlichungstag der Patentschrift :
**25.11.92 Patentblatt 92/48**

(21) Anmeldenummer : **90102350.7**

(22) Anmeldetag : **07.02.90**

(51) Int. Cl.⁵ : **C07C 227/40**

---

(54) Verfahren zur Gewinnung von wässrigen Lösungen freier neutraler alpha-Aminosäuren aus wässrigen Lösungen ihrer Alkalimetallsalze.

(30) Priorität : **21.02.89 DE 3905275**

(43) Veröffentlichungstag der Anmeldung :
**29.08.90 Patentblatt 90/35**

(45) Bekanntmachung des Hinweises auf die
Patenterteilung :
**25.11.92 Patentblatt 92/48**

(84) Benannte Vertragsstaaten :
**AT BE CH DE ES FR GB IT LI NL**

(56) Entgegenhaltungen :
**US-A- 2 700 054**
**US-A- 3 330 749**

(73) Patentinhaber : **DEGUSSA AG**
**Weissfrauenstrasse 9**
**W-6000 Frankfurt (Main) (DE)**

(72) Erfinder : **Hasselbach, Hans-Joachim, Dr.**
**Ahornweg 11**
**W-6458 Rodenbach 1 (DE)**
Erfinder : **Kleemann, Axel, Prof.**
**Bornweg 36**
**W-6052 Mühleim (DE)**
Erfinder : **Klenk, Herbert, Dr.**
**Gleiwitzerstrasse 21**
**W-6450 Hanau am Main 9 (DE)**
Erfinder : **Weigel, Horst**
**Odenwaldstrasse 56**
**W-6458 Rodenbach (DE)**

## Beschreibung

Gegenstand der Erfindung ist ein Verfahren zur Gewinnung von wäßrigen Lösungen freier neutraler $\alpha$-Aminocarbonsäuren aus wäßrigen Lösungen ihrer Alkalimetallsalze mittels eines stark sauren Kationenaustauschers in der $H^+$-Form.

Wäßrige Lösungen von Alkalimetallsalzen von neutralen $\alpha$-Aminocarbonsäuren fallen beispielsweise bei der alkalischen Hydrolyse der entsprechenden $\alpha$-Aminonitrile an.

Die Gewinnung von freien neutralen $\alpha$-Aminocarbonsäuren aus wäßrigen Lösungen ihrer Alkalimetallsalze mittels eines stark sauren Kationenaustauschers in der $H^+$-Form ist an sich bekannt (vgl. z.B. Greenstein and Winitz, Chemistry of the Amino Acids, Wiley, New York and London 1961, Seite 1459 ff.). Dabei werden die Alkalimetall-Kationen und die $\alpha$-Aminocarbonsäure an den Ionenaustauscher gebunden. Nach Auswaschen des Ionenaustauschers mit Wasser wird die $\alpha$-Aminocarbonsäure mit einer verdünnten wäßrigen Ammoniaklösung eluiert. Beim Eindampfen des Eluats wird das Ammoniak ausgetrieben und kann gegebenenfalls zurückgewonnen werden. Die freie $\alpha$-Aminocarbonsäure kann aus dem Rückstand isoliert werden. Dieses bekannte Verfahren weist jedoch mehrere Nachteile auf: Da die Alkalimetall-Kationen und die $\alpha$-Aminocarbonsäure an den Ionenaustauscher gebunden werden, steht dessen nutzbare Kapazität für die Bindung der Alkalimetall-Kationen bestenfalls zur Hälfte zur Verfügung. Es wird Ammoniak als zusätzlicher Hilfsstoff benötigt. Und schließlich sind die Eluate im Vergleich mit den Ausgangslösungen stark verdünnt, und es müssen daher, insbesondere bei leicht wasserlöslichen $\alpha$-Aminocarbonsäuren, wie Glycin, Alanin oder $\alpha$-Aminobuttersäure, beträchtliche Wassermengen verdampft werden.

Das erfindungsgemäße Verfahren ist nun dadurch gekennzeichnet, daß man zwei gleich große Austauschersäulen einsetzt, den Auslauf der ersten Säule mit dem Kopf der zweiten Säule verbindet, auf den Kopf der ersten Säule zunächst die Lösung des Alkalimetallsalzes aufgibt, anschließend durch Aufgabe von Wasser die um die am Austauscher adsorbierten Alkalimetall-Kationen verminderte Restlösung auf die zweite Säule verdrängt, am Auslauf der zweiten Säule zunächst einen produktfreien Vorlauf abtrennt, und, wenn im Ablauf aus der zweiten Säule freie $\alpha$-Aminocarbonsäure auszutreten beginnt, die Vorlage wechselt und auf den Kopf der ersten Säule weiter solange Wasser aufgibt, bis sich in der Vorlage ein Misch-pH-Wert zwischen 5 und 7 eingestellt hat, die bisher erste Säule abkoppelt und regeneriert, die bisher zweite Säule als nunmehr erste Säule an eine frisch regenerierte Säule als zweite Säule ankoppelt und den ganzen Vorgang beliebig oft wiederholt.

Vorzugsweise wird nach dem Wechsel der Vorlage auf den Kopf der ersten Säule solange Wasser aufgegeben bis sich in der Vorlage ein Misch-pH-Wert eingestellt hat, der dem isoelektrischen Punkt der jeweiligen $\alpha$-Aminocarbonsäure entspricht.

Die Vorteile des erfindungsgemäßen Verfahrens liegen darin, daß kein zusätzliches Eluationsmittel benötigt wird, und daß die Produktlösungen im Vergleich mit den Ausgangslösungen nicht verdünnt, sondern sogar aufkonzentriert werden. Der Verlust an $\alpha$-Aminocarbonsäure liegt deutlich unter 5 % und beträgt in der Regel höchstens 2 %.

Zur praktischen Durchführung des erfindungsgemäßen Verfahrens werden zwei gleich große und gleich dimensionierte Austauschersäulen in der Weise hintereinander geschaltet, daß der Auslauf der ersten Säule A mit dem Kopf der zweiten Säule B verbunden ist. Als Kationenaustauscher eignen sich alle handelsüblichen stark sauren Ionenaustauscherharze, insbesondere solche auf der Grundlage von mit Divinylbenzol vernetztem und mit $HO_3S$-Gruppen funktionalisiertem Polystyrol.

Auf den Kopf der Säule A wird zunächst die Lösung des Alkalimetallsalzes und dann Wasser aufgegeben. In der Lösung des Alkalimetallsalzes soll zweckmäßigerweise das Äquivalentverhältnis von Alkalimetall-Kationen zu der $\alpha$-Aminocarbonsäure zwischen 0,9 bis 1,5 : 1 vorzugsweise zwischen 1,1 bis 1,3 : 1 liegen. Es ist auch zweckmäßig, wenn die Lösung außer den Carboxylationen der $\alpha$-Aminocarbonsäure und Hydroxylionen andere Anionen, wie Carbonat-, Hydrogencarbonat-, Halogenid- oder Sulfationen, höchstens in Mengen von 0,1 Äquivalent, bezogen auf den Gehalt an $\alpha$-Aminocarbonsäure, enthält.

Die Konzentration der Lösung des Alkalimetallsalzes unterliegt nach unten keiner Beschränkung, denn der Effekt der Aufkonzentrierung tritt umso stärker hervor, je verdünnter die Ausgangslösung ist. Nach oben kann sich unter Umständen eine Beschränkung insofern ergeben, als durch den Effekt der Aufkonzentrierung nicht das Löslichkeitsprodukt der jeweiligen $\alpha$-Aminocarbonsäure überschritten werden sollte.

Während der Aufgabe der Lösung des Alkalimetallsalzes und der anschließenden Verdrängung mit Wasser läuft am Auslauf der Säule B zuerst ein produktfreier Vorlauf ab. Zunächst in der Säule A, anschließend auch in der Säule B wird erst einmal neben den Alkalimetall-Kationen auch die $\alpha$-Aminocarbonsäure gebunden. Bei weiterer Aufgabe von Ausgangslösung wird die $\alpha$-Aminocarbonsäure durch nachfolgende Alkalimetall-Kationen verdrängt. Das hat zur Folge, daß in der jeweiligen Zone des Harzbettes, in der sich dieser Verdrängungsvorgang abspielt, die Konzentration an $\alpha$-Aminocarbonsäure über die in der Ausgangslösung ansteigt. Die Front der Alkalimetall-Kationen schiebt nun stetig die Zone erhöhter Konzentration an $\alpha$-Aminocarbonsäure vor sich her, zunächst durch die Säule A, anschließend auch durch die Säule B. Schließlich beginnt im Ablauf der Säule B freie α-Aminocarbonsäure auszutreten. Zu erkennen ist dies daran, daß sich in der Vorlage Schlieren bilden, weil sich die Dichte des Ablaufes deutlich erhöht. Auch die Leitfähigkeit des Ablaufes ändert sich signifikant.

Die Menge an Ausgangslösung und die Menge an Verdrängungswasser müssen so aufeinander abgestimmt sein, daß zwei wesentliche Bedingungen erfüllt werden. Zum einen soll am Auslauf von Säule B eine α-Aminocarbonsäure-Lösung mit einem pH-Wert zwischen 5 und 7 anfallen, und zwar in einer solchen Menge, daß eine möglichst vollständige Nutzung der Austauschkapazität einer Säule erreicht wird. Zum anderen soll die Säule A durch das Verdrängungswasser gleichzeitig so weitgehend von Ausgangslösung befreit sein, daß sie unmittelbar danach regeneriert werden kann.

Als Produkt-Vorlage wird zweckmäßigerweise ein Gefäß eingesetzt, das mit einer Mischeinrichtung, im einfachsten Falle einem Rührer, und mit einer Vorrichtung zur pH-Messung ausgerüstet ist. Das Ende der Produktfraktion wird dadurch angezeigt, daß sich in der Produkt-Vorlage ein Misch-pH-Wert zwischen 5 und 7, vorzugsweise der dem isoelektrischen Punkt der jeweiligen α-Aminocarbonsäure entsprechende pH-Wert eingestellt hat.

Die Säule A ist jetzt vollständig mit Alkalimetall-Kationen beladen und auch bereits mit Wasser ausgewaschen. Sie wird von der Säule B abgekoppelt und in an sich bekannter Weise mit einer verdünnten Mineralsäure regeneriert, mit Wasser säurefrei gewaschen und rückgespült.

Die Säule B dagegen ist noch keineswegs vollständig mit Alkalimetall-Kationen beladen und wird daher nun für einen weiteren Zyklus als erste Säule eingesetzt und an die regenerierte Säule A als zweite Säule so angekoppelt, daß wieder der Auslauf der ersten Säule (B) mit dem Kopf der zweiten Säule (A) verbunden ist.

Um die Wartezeit während der Regenerierung der Säule A einzusparen, ist es vorteilhaft, wenn mehr als zwei Säulen vorhanden sind, so daß bei Bedarf immer eine frisch regenerierte Säule zur Verfügung steht, die als zweite Säule eingesetzt werden kann. Dadurch wird letztlich eine quasi-kontinuierliche Durchführung des erfindungsgemäßen Verfahrens ermöglicht.

Bei der Bestimmung der Aufgabemenge muß unterschieden werden zwischen der Anfahrphase, in der zwei frisch regenerierte Austauschersäulen zur Verfügung stehen, und den nachfolgenden Produktions-Zyklen, in denen immer nur eine frisch regenerierte Austauschersäule und eine bereits teilweise beladene Austauschersäule zur Verfügung steht.

Die Aufgabemenge in der Anfahrphase ($V_{Start}$) kann für eine gegebene Austauscheranlage und eine gegebene Ausgangslösung annähernd errechnet werden nach der Gleichung

$$V_{Start} = (A \times V_H \times K : Me^+_{eff}), \quad (1)$$

wobei A einen Wert zwischen 2,3 und 2,7, vorzugsweise von 2,5, hat. Weiterhin bedeuten $V_H$ das Volumen des Harzbettes in einer Austauschersäule, K die nutzbare Kapazität des Harzes in Mol Alkalimetall-Kationen pro Liter Harz, $M^+_{eff}$ die effektiv auszutauschende Menge an Alkalimetallionen, um einen Misch-pH-Wert von 6 in der Produktfraktion zu erreichen, in Mol Alkalimetall-Kationen pro Liter Ausgangslösung.

$V_H$ und K werden jeweils an dem Harz in der Na$^+$-Form bestimmt, $V_H$ am mit Wasser überschichteten Harz, und K durch acidimetrische Titration. $M^+_{eff}$ kann in einem Vorversuch durch acidimetrische Titration bestimmt werden.

Analog kann die Aufgabemenge in den nachfolgenden Produktions-Zyklen annähernd errechnet werden nach der Gleichung

$$V_{Zykl} = (V_H \times K : Me^+_{eff}), \quad (2)$$

in der $V_{Zykl}$ das Aufgabevolumen für die Lösung des Alkalimetallsalzes bedeutet und K und $Me^+_{eff}$ wieder die bereits genannte Bedeutung haben. Das bedeutet, daß in den Produktions-Zyklen die Aufgabemenge so bemessen ist, daß die Austauschkapazität einer Säule voll genutzt wird.

Die Erfindung wird durch die nachfolgenden Beispiele näher erläutert:

Beispiel 1:

Eingesetzt wurde eine aus zwei Säulen bestehende Austauscheranlage mit einem stark sauren Ionenaustauscher in der H$^+$-Form (Lewatit® SP 112 von der Bayer AG). Das Volumen jedes der beiden Harzbetten betrug 13 l, gemessen an dem mit Wasser überschichteten Harz in der Na$^+$-Form. Das Verhältnis von Querschnitt zu Höhe des Harzbettes betrug jeweils 1 : 6, die nutzbare Kapazität des Harzes 1,5 Mol Na$^+$/l.

Mit einer Pumpgeschwindigkeit von 60 l/Stunde wurden zunächst eine durch Hydrolyse von Aminoacetonitril mit Natronlauge hergestellte Ausgangslösung folgender Zusammensetzung

1,3 Mol/l Glycin
0,1 Mol/l Iminodiessigsäure (Nebenprodukt)
1,7 Mol/l Na$^+$

in einer Menge von 30,5 l und anschließend 13,0 l Wasser auf den Kopf der ersten Säule aufgegeben. Am Ablauf der zweiten Säule fielen währenddessen zunächst 37,0 l produktfreier Vorlauf an. Nun wurde die Vorlage gewechselt und eine Produktfraktion gesammelt, bis sich in der Vorlage ein Misch-pH-Wert von 6 einstellte. Das Volumen der Produktfraktion betrug 6,5 l.

Dann wurde die erste Säule abgekoppelt und der Regenerierung zugeführt. Die bisher zweite Säule wurde als nunmehr erste Säule mit einer frisch regenerierten Säule als zweiter Säule verbunden. Damit war die Anfahrphase beendet und die eigentlichen Produktions-Zyklen konnten beginnen.

Auf den Kopf der ersten Säule wurden zunächst 12,2 l der obigen Ausgangslösung und anschließend 13,0 l Wasser aufgegeben. Am Ablauf der zweiten Säule fielen währenddessen zunächst 18.7 l produktfreier Vorlauf an. Nun wurde wieder die Vorlage gewechselt und eine Produktfraktion gesammelt, bis sich in der Vorlage ein Misch-pH-Wert von 6 einstellte. Das Volumen der Produktfraktion betrug 6,5 l.

Damit war der erste Produktions-Zyklus beendet. Er wurde in genau gleicher Weise noch siebenmal wiederholt. Insgesamt wurden während der acht Produktions-Zyklen 52,0 l Produktlösung folgender Zusammensetzung

2,40    Mol/l Glycin

0,18    Mol/l Iminodiessigsäure

0,18    Mol/l $Na^+$

erhalten. Dies entspricht einer Rückgewinnungsrate für das Glycin von 98 %.

Beispiel 2:

In der gleichen Austauscheranlage wie im Beispiel 1 wurde eine höher konzentrierte Ausgangslösung folgender Zusammensetzung

2,00    Mol/l Glycin

0,20    Mol/l Iminodiessigsäure

2,60    Mol/l $Na^+$

aufgearbeitet.

In der Anfahrphase wurden 24,4 l dieser Lösung und 13,0 l Wasser aufgegeben. Im ersten nachfolgenden Produktions-Zyklus wurden 8,1 l Ausgangslösung und 13,0 l Wasser aufgegeben. Es fielen 14,35 l produktfreier Vorlauf und 6,75 l Produktlösung an.

In insgesamt acht Produktions-Zyklen wurden 54,0 l Produktlösung folgender Zusammensetzung

2,35    Mol/l Glycin

0,24    Mol/l Iminodiessigsäure

0,24    Mol/l $Na^+$

erhalten, was wiederum einer Rückgewinnungsrate für das Glycin von 98 % entspricht.

Ein Vergleich der Beispiele 1 und 2 zeigt, daß der Effekt der Aufkonzentrierung umso beträchtlicher ist, je verdünnter die Ausgangslösung ist.

Beispiel 3:

Eingesetzt wurde eine aus zwei Säulen bestehende Austauscheranlage mit dem gleichen Harz wie in Beispiel 1. Das Volumen jedes der beiden Harzbetten betrug 0,5 l, wiederum gemessen an dem mit Wasser überschichteten Harz in der $Na^+$-Form. Das Verhältnis von Querschnitt zu Höhe des Harzbettes betrug jeweils ebenfalls 1 : 6.

Mit einer Pumpgeschwindigkeit von 2 l/Stunde wurde eine Ausgangslösung folgender Zusammensetzung*

1,2    Mol/l D,L-$\alpha$-Aminobuttersäure

1,3    Mol/l $Na^+$

aufgearbeitet.

In der Anfahrphase wurden 1 440 ml dieser Lösung und 500 ml Wasser aufgegeben. Es wurden 1 515 ml produktfreier Vorlauf und 425 ml Produktlösung erhalten. Im ersten nachfolgenden Produktions-Zyklus wurden 575 ml Ausgangslösung und 500 ml Wasser aufgegeben. Es fielen 650 ml produktfreier Vorlauf und 425 ml Produktlösung an.

In insgesamt zehn Produktions-Zyklen wurden 4,25 l Produktlösung mit einem Gehalt von 1,60 Mol/l D,L-$\alpha$-Aminobuttersäure und unter 0,1 Mol/l $Na^+$ erhalten. Die Rückgewinnungsrate für die D,L-$\alpha$-Aminobuttersäure betrug 98 %.

Beispiel 4:

In der gleichen Austauscheranlage wie im Beispiel 3 wurde eine Ausgangslösung der folgenden Zusammensetzung

1,00    Mol/l D,L-Alanin

1,10    Mol/l $Na^+$

aufgearbeitet.

In der Anfahrphase wurden 1 705 ml dieser Lösung und 500 ml Wasser aufgegeben. Es wurden 1 730 ml produktfreier Vorlauf und 475 ml Produktlösung erhalten. Im ersten nachfolgenden Produktions-Zyklus wurden 680 ml Ausgangslösung und 500 ml Wasser aufgegeben. Es fielen 705 ml produktfreier Vorlauf und 475 ml Produktlösung an.

In insgesamt zehn Produktions-Zyklen wurden 4,75 l Produktlösung mit einem Gehalt von 1,40 Mol/l D,L-Alanin und unter 0,10 Mol/l $Na^+$ erhalten. Die Rückgewinnungsrate für das D,L-Alanin betrug 98 %.

**Patentansprüche**

1. Verfahren zur Gewinnung von wäßrigen Lösungen freier neutraler $\alpha$-Aminocarbonsäuren aus wäßrigen Lösungen ihrer Alkalimetallsalze mittels eines stark sauren Kationenaustauschers in der $H^+$-Form,
dadurch gekennzeichnet,
daß man zwei gleich große Austauschersäulen einsetzt, den Auslauf der ersten Säule mit dem Kopf der zweiten Säule verbindet, auf den Kopf der ersten Säule zunächst die Lösung des Alkalimetallsalzes aufgibt, anschließend durch Aufgabe von Wasser die um die am Austauscher adsorbierten Alkalimetall-Kationen verminderte Restlösung auf die zweite Säule verdrängt, am Auslauf der zweiten Säule zunächst einen produktfreien Vorlauf abtrennt und, wenn im Ablauf aus der zweiten Säule freie $\alpha$-Aminocarbonsäure auszutreten beginnt, die Vorlage wechselt und auf den Kopf der ersten Säule weiter solange Wasser aufgibt, bis sich in der Vorlage ein Misch-pH-Wert zwischen 5 und 7 eingestellt hat, die bisher erste Säule abkoppelt und regeneriert, die bisher zweite Säule als nunmehr erste Säule an eine frisch regenerierte Säule als zweite Säule ankoppelt und den ganzen Vorgang beliebig oft wiederholt.

2. Verfahren nach Anspruch 1,
dadurch gekennzeichnet,
daß man nach Wechsel der Vorlage auf den Kopf der ersten Säule solange Wasser aufgibt, bis sich in der Vorlage ein Misch-pH-Wert eingestellt hat, der dem isoelektrischen Punkt der jeweiligen α-Aminocarbonsäure entspricht.

## Claims

1. A process for the recovery of aqueous solutions of free neutral α-aminocarboxylic acids from aqueous solutions of their alkali metal salts by means of a strongly acid cation exchanger in the $H^+$-form, characterised in that two exchanger columns of equal size are used, the outflow from the first column is connected to the head of the second column, the solution of the alkali metal salt is first supplied to the head of the first column and the remaining solution diminished by the alkali metal cations adsorbed on the exchanger is then displaced to the second column by the addition of water, a product-free first runnings is first discharged at the outflow of the second column, and the receiver is replaced when free α-aminocarboxylic acid begins to be discharged in the outflow from the second column, and water continues to be supplied to the head of the first column until a mixed pH of from 5 to 7 is established in the receiver, the previously first column is disconnected and regenerated, the previously second column is now connected as first column to a freshly regenerated column which is now the second column, and the whole process is repeated any number of times.

2. A process according to claim 1, characterised in that after replacement of the receiver, water continues to be supplied to the head of the first column until a mixed pH value corresponding to the isoelectric point of the given amino acid is established in the receiver.

## Revendications

1. Procédé d'obtention de solution aqueuses d'acides α-aminés neutres, libres, à partir de solutions aqueuses de leurs sels de métal alcalin, à l'aide d'un échangeur de cations fortement acide sous la forme $H^+$, procédé caractérisé en ce que l'on met en oeuvre deux colonnes d'échange de même grandeur que l'on relie l'écoulement de la première colonne à la tête de la seconde colonne, que l'on verse sur la tête de la première colonne en premier lieu la solution de sel de métal alcalin, que l'on déplace ensuite par addition d'eau la solution restante, diminuée des cations de métal alcalin absorbés sur l'échangeur, que l'on sépare à l'écoulement de la seconde colonne en premier lieu un écoulement sans produit et quand à l'écoulement à partir de la seconde colonne les acides α-aminés commencent à sortir, on change le récipient-collecteur et on verse sur la tête de la première colonne à nouveau de l'eau aussi longtemps que dans le récipient-collecteur une valeur de pH du mélange s'ajuste entre 5 et 7, que l'on débranche la colonne jusqu'ici la première et qu'on la régénère, que l'on couple la colonne jusqu'ici la seconde, en tant que désormais première colonne, à une colonne fraîchement régénérée comme deuxième colonne et que l'on répète la totalité du processus aussi souvent que l'on voudra.

2. Procédé selon la revendication 1, caractérisé en ce que l'on verse après échange du récipient-collecteur de l'eau sur la tête de la première colonne aussi longtemps qu'une valeur de pH du mélange dans le récipient-collecteur ait été ajustée qui corresponde au point iso-électrique de l'acide α-aminé correspondant.